# EUROPEAN PATENT SPECIFICATION

(11) **EP 2 367 784 B1**
(45) Date of publication and mention of the grant of the patent: **02.01.2013**
(21) Application number: 09834229.8
(22) Date of filing: 11.06.2009
(51) Int. Cl.: A61K 31/122, A61P 35/00

(54) **BICYCLIC TRITERPENOID IRIPALLIDAL AS A NOVEL ANTIGLIOMA AND ANTINEOPLASTIC THERAPY**
BIZYKLISCHES TRITERPENOIDES IRIPALLIDAL ALS NEUARTIGE ANTIGLIOM UND ANTINEOPLASTISCHE THERAPIE
IRIPALLIDAL TRIPERPÉNOÏDE BICYCLIQUE EN TANT QUE NOUVEAU TRAITEMENT ANTI-GLIOME ET ANTINÉOPLASIQUE

(30) Priority: 22.12.2008 IN DE29152008
(43) Date of publication of application: 28.09.2011
(73) Proprietor: Department of Biotechnology, New Delhi 11 0003 (IN); National Brain Research Centre, Manesar 122 050 (IN)
(72) Inventor: SEN, Ellora, Haryanal 220 050 (IN); KOUL, Nitin, Haryana 122 050 (IN); SHARMA, Vivek, Haryana 122 050 (IN); TEWARI, Richa, Haryana 122 050 (IN)
(74) Representative: Thomas, Simon
(86) International application number: PCT/IN2009/000336
(87) International publication number: WO 2010/073258

(56) References cited:
- WO-A1-97/36604
- WO-A2-2008/100449
- KOUL NITIN ET AL: "Bicyclic triterpenoid Iripallidal induces apoptosis and inhibits Akt/mTOR pathway in glioma cells.", BMC CANCER, vol. 10, 328, 2010, pages 1-8, XP002672778, ISSN: 1471-2407
- DATABASE CAPLUS 2009 SHAO, LEI ET AL: 'Iridals Are a Novel Class of Ligands for Phorbol Ester Receptors with Modest Selectivity for the RasGRP Receptor Subfamily', XP008139246 Retrieved from STN Database accession no. 2001:739610 & JOURNAL OF MEDICINAL CHEMISTRY vol. 44, no. 23, 2001, pages 3872 - 3880

## Description

### FIELD OF INVENTION

This invention relates to Bicyclic triterpenoid Iripallidal as a novel anti-glioma and anti-neoplastic therapy *in vitro*.

### BACKGROUND OF INVENTION

Glioblastoma multiformes (GBM) represents one of the most malignant and prevalent brain tumor that still remains incurable. Despite recent advances in understanding molecular mechanisms involved in GBM progression, the prognosis of the most malignant brain tumor continues to be dismal. Iripallidal [(-) (6R, 10S, 11S, 18R, 22S) -26-Hydroxy-22-α-methylcycloirid-16-enal NSC 631939] is bicyclic triterpenoid that displays anti-proliferative activity in a NCI 60 cell line screen both at micromolar to nanomolar range. It binds to RasGRP3, a phorbol ester receptor that links DAG/phorbol ester signalling with Ras activation. It also induces phosphorylation of ERK1/2 in a Ras dependent manner.

Ras proteins are members of a super family of related small GTPases implicated in cellular proliferation and transformation. Although Ras is usually associated with cell proliferation; growth arrest, senescence or apoptosis in response to activated Ras has also been reported. Ras not only differentially regulates discrete cell death programs through Ras/PKC mediated, but it also induces non-apoptotic cell death in GBM. Proapoptotic effects of Ras are mediated by the MAPK pathway. The major MAPK families include extracellular signal related kinases (ERK), c-Jun N-terminal kinases (JNK) and the p38MAPK. The alkyl phospholipid Perifosine inhibits Ras/ERK signaling pathways to induce apoptosis in gliomas. Besides, ERK activation plays an active role in mediating cisplatin-induced apoptosis of HeLa cells. As Ras activation occurs in GBMs, this high level of active Ras has been a target for Ras inhibitors mediated glioma therapy. We therefore evaluated the ability of Iripallidal to induce cell death by modulating Ras.

Natural products continue to be an important source of chemotherapeutic agents. The plant-derived product is paclitaxel (currently marketed as TAXOL ® by Bristol-Myers Squibb Oncology Division) is a classic example of natural products derived chemotherapeutic. Paclitaxel is a natural tubulin binding diterpene product isolated from *Taxus brevafolia,* inhibiting cancer cell and is an effective as chemotherapy for several types of neoplasms.

As both Paclitaxel and Iripallidal belong to the terpenoid family and Inventors observed an anti-proliferative effect of Iripllidal on GBM cell lines, Inventors investigated the effect of Iripallidal on the viability of human breast, cervical, liver, colon and acute myeloid leukemia cancer cell lines.

There is no literature available on the chemotherapeutic effect of Iripallidal on GBM and other cancer cell lines. The unique property of the therapy with Iripallidal is the killing of a wide range of human cancer cell lines without affecting normal human monocytes, *in vitro.*

There is no literature available regarding the use of Iripallidal as a therapeutic measure for treatment of glioblastoma, breast, colon, cervical, hepatocellular and cancer of the myeloid origin.

Extensive in vitro screening of compounds with anticancer activity performed by National Cancer Institute identified more than 70,000 compounds for their antiproliferation activities against a panel of 60 human cancer cell lines. Autodock program revealed that the **Iridals** dock to the same position on protein kinase C delta as do the phorbol esters. Biological analysis of two iridals, NSC 631939 and NSC 631941, revealed that they bound to (i) protein kinase C alpha (ii) RasGRP3, a phorbol ester receptor that directly links diacylglycerol/phorbol ester signaling with Ras activation. Both compounds induced phosphorylation of ERK1/2, a downstream indicator of Ras activation.

Besides this nothing is known regarding the effect of Iripallidal on the proliferation of human glioblastoma, breast, colon, cervical, hepatocellular and cancer of the myeloid origin *in vitro.*

### OBJECTS OF THE INVENTION

The main object is to identify iripallidal as a new chemo-therapeutic agent against glioblastoma multiforme.

Other object is to identify the ability of Tripallidal to inhibit proliferation of human breast, colon, cervical, hepatocellular and myeloid leukemic cancer cell lines.

Another object is to overcome the nonspecific chemotherapy, which adversely affects normal cells.

Yet another object is to identify iripallidal wherein it decreases the proliferation and viability of several human cancer cell. lines without effecting normal cells.

### STATEMENT OF INVENTION

This invention relates to a bicyclic triterpenoid Iripallidal as a novel anti-glioma and anti-neoplastic agent *in vitro*, having the composition [(-)-(6R, 10S,11S,18R,22S)-26-Hydroxy-22-α methylcyloirid-16-enal NSC 631939], wherein it binds to Ras GRP3, a phorbol ester receptor that links DAG/phorbol ester signaling with Ras activation and induces phosphorylation of ER K1/2 in a Ras dependent manner, reducing proliferation of cancer cell lines to the extent of 50-70%.

Specifically, the present invention provides iripallidal ((-)-(6R,10S,11S,18R,22S)-26-hydroxy-22α-methylcycloirid-16-enal) for use in the treatment of cancer, wherein the cancer is selected from glioblastoma multiforme, breast cancer, cervical cancer, liver cancer, acute myeloid leukaemia and colon cancer.

The invention also provides the use of iripallidal for the *in vitro* treatment of a glioma cell line selected from U87MG, A172, LN229 and T98G; the breast cancer cell line MCF7; the cervical cancer cell line HeLa; the hepatocellular carcinoma cell line HcpG2, the acute myeloid leukaemia cell line THP1 and the colon carcinoma cell line HT-29.

### BRIEF DESCRIPTION OF ACCOMPANYING DRAWINGS

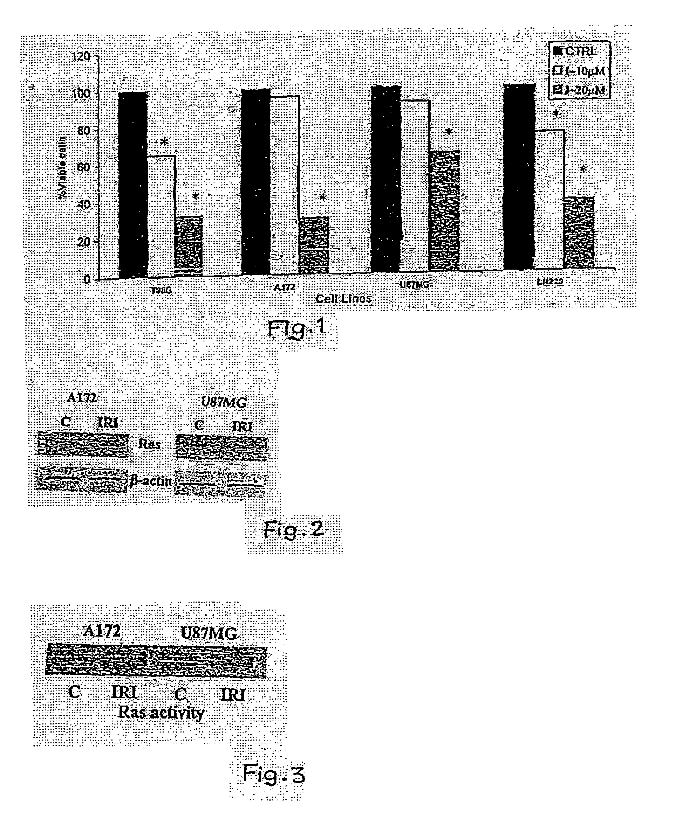

**Figure 1****.** Shows the anti- proliferative effect of Iripallidal on glioma cells. Viability of different glioma cell lines treated with Iripallidal was determined by MTS assay. The graph represents the percentage viable cells of control observed when U87MG, A172, LN229 and T98G cells were treated with different concentrations of Iripallidal for 24 hours. Ctrl-control; I-Iripallidal.
**Figure 2****.** Illustrates Western blot analysis demonstrating expression of Ras in A172 and U87MG cells treated with Iripallidal. A representative blot is shown from three independent experiments with identical results. Blots were reprobed for β actin to establish equivalent loading. C-control; IRI-Iripallidal 20µM.
**Figure 3****.** demonstrates Effect of Iripallidal on Ras activity. The level of Ras-GTP in protein extracts of control and Iripallidal treated glioma cells was determined by the ability of Ras-GTP to bind to a specific protein domain of Raf in the form of a GST-fusion protein. An increase Ras activity was observed in Iripallidal treated cells as compared to the control. The figure is representative from two independent experiments with identical results. C-control; IRI-Iripallidal 20µM.

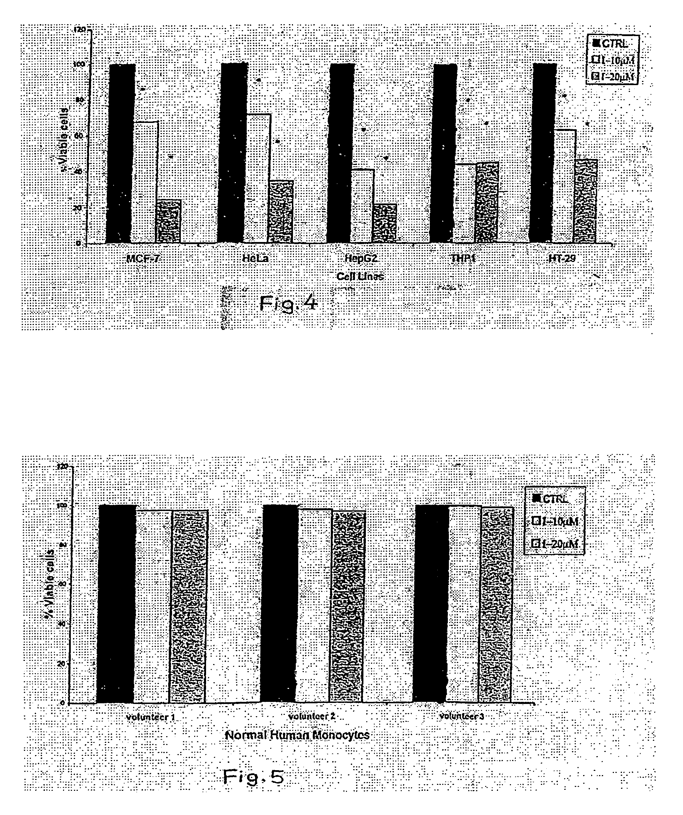

**Figure 4****.** depicts the anti-proliferative effect of Iripallidal on breast, cervical, hepatocellular carcinoma, acute myeloid leukemia and colon carcinoma cell lines. Viability of MCF-7 (breast), HeLa (cervical), HepG2 (hepatocellular carcinoma), THP1 (acute myeloid leukemia), HT-29 (colon carcinoma) cells treated with different doses of Iripallidal for 24 hours was determined by MTS assay. The graph represents the percentage viable cells of control observed when cells were treated with different concentrations of Iripallidal for 24 hours. Ctrl-control; I-Iripallidal.
**Figure 5****.** Shows iripallidal does not effect the viability of normal human monocytes.
   Graph depicts the effect of Iripallidal on normal human monocytes. Viability of monocytes treated with Iripallidal was determined by MTS assay. The graph represents the percentage viable cells of control observed when cells were treated with different concentrations of Iripallidal for 24 hours. Ctrl-control; I-Iripallidal.

### DETAILED DESCRIPTION OF THE INVENTION

GBM represents one of the most malignant brain tumors and there is no effective treatment for GBM. In addition to inhibiting glioblastoma cell proliferation, Iripallidal also inhibited the proliferation of breast, colon, cervical, liver and cancer of the myeloid origin.

Results indicate that human glioma cell lines are sensitive to the anti-proliferative effect of Iripallidal and Iripallidal increases both Ras levels and activity in glioma cell lines. These results raise the possibility that Iripallidal may prove effective in the treatment of GBM, by inhibiting its proliferation.

As both Paclitaxel and Iripallidal belong to the terpenoid family and we observed an anti-proliferative effect of Iripllidal on GBM cell lines, we investigated the effect of Iripallidal on the viability of human breast, cervical, liver, colon and acute myeloid leukemia cancer cell lines.

Iripallidal decreased the viability of glioblastoma cell lines significantly *in vitro.* Iripallidal elevated both Ras levels and Ras activity in GBM cell lines. Ras is known to regulate discrete cell death programs through Ras mediated and Fas mediated apoptotic pathways. It also induces non-apoptotic cell death in GBM.

Inventors have procured Iripallidal from Calbiochem, USA for all our experiments.

In another embodiment GBM cell lines U87MG, A172, T98G, and LN229 were treated with different concentration of Iripallidal (in Dimethyl sulphoxide, DMSO) in serum free media for 24 hours and cell viability was assessed using the [3-(4,5-dimethylthiazol-2-yl)-5-(3-carboxymethoxy-phenyl)-2-(4-sulfophenyl)- 2H-tetrazolium, inner salt] (MTS) assay. Values were expressed as percent cell viability relative to control.

In another embodiment, protein isolated from glioma cells were treated with Iripallidal and electrophoresed on 6% to 10% polyacrylamide gel, Western blotting was performed and expression of Ras determined as described previously.

In another embodiment the Ras activity was performed using a commercially available kit from Upstate Biotechnology (Temecula, CA, USA). Briefly, glioma cell lines (2X10⁶) treated with Iripallidal were lysed and the cleared lysates were incubated with bead-bound RAS binding domain of Raf-1 protein to precipitate Ras-GTP. The precipitates were subjected to SDS-PAGE and Western blotting. Staining of the blots with a Ras Ab revealed the level of Ras activation in the lysate.

In another embodiment colon cancer cell line HT29, breast cancer line MCF-7, cervical cancer cell line HeLa, hepatocellular carcinoma cell line HepG2 and acute myeloid leukemic cell line THP1 were treated with different concentration of Iripallidal (in Dimethyl sulphoxide, DMSO) in serum free media for 24 hours and cell viability was assessed using the MTS assay. Values were expressed as a percentage relative to those obtained in controls.

In another embodiment normal human monocytes were treated with different concentration of Iripallidal *in* serum free media for 24 hours and cell viability was assessed using the MTS assay. Values were expressed as a percentage relative to those obtained in controls.

The following examples are given by way of explanation and for illustration only and these examples should not be construed in any manner to limit the scope of invention.

### Examples

### Example 1: Cell culture and treatment

Glioblastoma cell lines U87MG, A172, T98G, and LN229; breast cancer cell line MCF7, cervical cancer cell line HeLa, hepatocellular carcinoma cell line HepG2, acute myeloid leukemia THP1, colon carcinoma cell line HT-29 and acute myeloid leukemic cell line THP1 were obtained from American Type Culture Collection and cultured in DMEM supplemented with 10% fetal bovine serum. On attaining semi-confluence, cells were switched to serum free media and after 12 hours, cells were treated with different concentration of Iripallidal (in DMSO) in serum free media for 24 hours. Following treatment, the cells were processed for Western blot analysis. DMSO treated cells were used as controls.

### Example 2: Preparation of peripheral blood mononuclear cells (PBMC) from normal human ex vivo.

Whole blood (5 ml) was drawn from normal human volunteers and mononuclear cells were separated by Histopaque density gradient centrifugation. Briefly, blood was diluted twice with phosphate buffered saline (PBS) and layered over Histopaque (Sigma, MO, USA) and centrifuged at 1500 rpm for 20 mins at room temperature. Peripheral blood mononuclear cells (PBMC) were collected carefully at the interface of Histopaque and plasma, diluted five times with PBS and centrifuged at 1500 rpm for 10 mins. PBMC thus pelleted were washed twice with PBS and seeded onto glass petri dishes and allowed to adhere for 3 hrs. Non adherent cells were removed by gentle washing with PBS twice and the adherent monocytes were used in MTS assay.

### Example 3: Determination of viability of glioblastoma, breast, liver, colon, myeloid leukemia cancer cell lines and normal human monocytes upon treatment with Iripallidal

Cell viability was assessed using the [3-(4,5-dimethylthiazol-2-yl)-5-(3-carboxymethoxy-phenyl)-2-(4-sulfophenyl)- 2H-tetrazolium, inner salt] (MTS) as described earlier. Following treatment of cancer cells (2×10³) and normal monocytes (10 ×10³) with different concentrations of Iripallidal for 24 hours in 96-well plates, 20 µl of MTS solution was added. After 4 hours of incubation the absorbance reflecting reduction of MTS by viable cells was determined at 490 nm. Values were expressed as a percentage relative to those obtained in controls.

### Example 4: Western Blot Analysis depicting increased Ras levels in Iripallidal treated glioma cells

Protein from whole cell lysates were isolated as described previously. Twenty to fifty µg of protein isolated from cells treated with Iripallidal was electrophoresed on 6% to 10% polyacrylamide gel. Western blotting was performed and Ras expression was determined as described. The blots were stripped and reprobed with anti-β-actin to determine equivalent loading.

### Example 5: Iripallidal increases Ras activity in glioma cells.

Effect of Iripallidal on the level of GTP-bound Ras. The level of Ras-GTP in protein extracts of control and Iripallidal treated glioma cells was determined by the ability of Ras-GTP to bind to a specific protein domain of Raf in the form of a GST-fusion protein. An increase in Ras activity was observed in Iripallidal treated cells as compared to the control. IRI denotes Iripallidal.

## Claims

1. Iripallidal ((-)-(6R,10S,11S,18R,22S)-26-hydroxy-22α-methylcycloirid-16-enal) for use in the treatment of cancer, wherein the cancer is selected from glioblastoma multiforme, breast cancer, cervical cancer, liver cancer, acute myeloid leukaemia and colon cancer.

2. Iripallidal for use according to claim 1, wherein the cancer is glioblastoma multiforme.

3. Use of Iripallidal for the *in vitro* treatment of a glioma cell line selected from U87MG, A172, LN229 and T98G; the breast cancer cell line MCF7; the cervical cancer cell line HeLa; the hepatocellular carcinoma cell line HepG2, the acute myeloid leukaemia cell line THP1 and the colon carcinoma cell line HT-29.

## Patentansprüche

1. Iripallidal ((-)-(6R,10S,11S,18R,22S)-26-Hydroxy-22α-methylcycloirid-16-enal) zur Verwendung bei der Behandlung einer Krebserkrankung, wobei die Krebserkrankung aus Glioblastoma multiforme, Brustkrebs, Gebärmutterhalskrebs, Leberkrebs, akuter myeloischer Leukämie und Dickdarmkrebs ausgewählt ist.

2. Iripallidal zur Verwendung nach Anspruch 1, wobei die Krebserkrankung Glioblastoma multiforme ist.

3. Verwendung von Iripallidal zur In-vitro-Behandlung einer Gliomzelllinie, die aus U87MG, A172, LN229 und T98G ausgewählt ist; der Brustkrebszelllinie MCF7; der Gebärmutterhalskrebszelllinie HeLa; der Leberzellkarzinomzelllinie HepG2, der akuten myeloischen Leukämiezelllinie THP1 und der Dickdarmkrebszelllinie HT-29.

## Revendications

1. Iripallidal ((-)-(6R,10S,11S,18R,22S)-26-hydroxy-22α-methylcycloirid-16-énal) destiné à être utilisé dans le traitement d'un cancer choisi parmi le glioblastome multiforme, le cancer du sein, le cancer du col de l'utérus, le cancer du foie, la leucémie myéloïde aiguë et le cancer du côlon.

2. Iripallidal destiné à être utilisé selon la revendication 1, dans laquelle le cancer est le glioblastome multiforme.

3. Utilisation de l'iripallidal pour le traitement in vitro d'une lignée cellulaire gliomale choisie des lignées U87MG, A172, LN229 et T98G ; la lignée cellulaire de cancer du sein MCF7 ; la lignée cellulaire de cancer du col de l'utérus HeLa ; la lignée cellulaire de carcinome hépatocellulaire HepG2 ; la lignée cellulaire de leucémie myéloïde aiguë THP1 ; et la lignée cellulaire de carcinome du côlon HT-29.
